# EUROPEAN PATENT APPLICATION

(11) **EP 0 771 877 A1**
(43) Date of publication of application: **07.05.1997**
(21) Application number: 95919640.3
(22) Date of filing: 25.05.1995
(51) Int. Cl.: C12N 15/62, C07K 14/71, G01N 33/566, C12P 21/02, A61K 38/17

(54) **GENE CODING FOR MODIFIED BONE MORPHOGENIC PROTEIN RECEPTOR**

(30) Priority: 26.05.1994 JP 137935/94
(71) Applicant: NIPPON MEAT PACKERS, INC., Osaka-shi, Osaka 541 (JP); Ueno, Naoto, Sapporo-shi, Hokkaido 065 (JP)
(72) Inventor: UENO, Naoto Hokko-komuinsyukusya 1-101, Sapporo-shi Hokkaido 065 (JP); NATSUME, Toru Nippon Meat Packers, Inc., Tsukuba-shi Ibaraki 300-26 (JP)
(74) Representative: Kolb, Helga, Dr. Dipl.-Chem.
(86) International application number: JP9501009
(87) International publication number: WO9533058

(57) **Abstract**

A gene coding for a modified bone morphogenic protein (BMP) receptor; a modified BMP receptor expressed by means of the gene; and the use of the modified receptor. As the modified BMP receptor binds specifically with BMP, it can widely be utilized as a reagent for purifying BMP, a diagnostic reagent for various diseases related to BMP, research reagent, medicines, and the like.

## Description

### TECHNICAL FIELD

This invention relates to a gene coding a modified bone-morphogenetic-protein receptor, to the modified bone-morphogenetic-protein receptor expressed by the gene, and to application of the receptor. More particularly, the invention relates to the gene coding a modified receptor comprising an extracellular domain of bone-morphogenetic-protein receptor, to the modified bone-morphogenetic-protein receptor expressed by the gene and to the receptor useful for diagnosing various bone-related diseases, screening for substances associated with bone production, purifying bone morphogenetic protein, and so forth.

### BACKGROUND OF THE INVENTION

With increases in aged people and changes in dietary habits, such diseases caused by bone disorders as osteoporosis, osteomalacia, osteosclerosis, osteoncus, failure of fracture healing and the like have increased. Therefore, research on and development of drugs and therapeutics of these bone disorders have been carried out.

Bone is referred to as a dynamic organ in which bone production (osteogenesis) and bone resorption are repeatedly carried out throughout one's life. Osteogenesis takes place in osteoblasts derived from mesenchymal cells, whereas bone resorption in osteoclasts from hematopoietic cells. By a so-called remodeling mechanism, old bone is replaced by new one, so that intensity of the bone is maintained as a whole. Bone at a resting stage is resorbed by the osteoclasts, and then the resorbed bone is repaired by the osteoblasts.

Various growth factors affecting such a series of bone remodeling processes are known. They are classified into two groups; one is a bone inducible factor making immature cells transform to osteoblasts and the other is an osteogenetic factor making osteoblasts produce bone. For example, retinoic acid may belong to the former and TGF-β to the latter. There is a growth factor named bone morphogenetic protein (a so-called osteogenetic factor, abbreviated to BMP) assumably possessing both characteristics of the bone-inducing and -producing factors. Expression of mRNA of BMP has been confirmed in such osseous tissues as tooth and bone as well as such organs as the heart, brain, lung, liver, kidney, skin and hair root. The expression of BMP mRNA suggested that BMP plays important roles in the epithelium-mesenchymal interaction in developmental phases of tissue construction. Other biological functions of the mRNA of BMP have attracted attention. On a protein level, BMP has been confirmed in such tissues as bone, tooth and osteosarcoma.

As for application of BMP, there may be mentioned, for example, a bone-production-inducible and a bone- and cartilage-production-inducible composition comprising BMP as an active component (Japanese Patent National Publications Nos. 500241/1990, 503649/1991 and 505098/1991), a bone-production-inducible drug to prevent and treat reduced-bone diseases (Japanese Patent laid-open No. 132426/1993), a bone- production-inducible polypeptide identified in regenerated bone marrow (Japanese Patent laid-open No. 282396/1990) and a usage of BMP for bone repair by combined use of BMP and TGF-β (Japanese Patent National Publication No. 505404/1993). With BMP, drugs for a purpose of topical administration are being developed at present. Moreover, there have long been needs for clinical application of BMP to promote fracture healing, bone-defect repair and treatment of reduced-bone diseases like osteoporosis, and needs for application of BMP as a novel tumor marker of osteosarcoma and as a remedy of BMP-causing tumors, those caused by BMP and oral diseases.

In 1960s, the name BMP was given to a proteinaceous molecule in bone that has a capacity to induce heterotopic osteogenesis. In 1989, the DNA sequence of BMP was confirmed and BMP was proved to be a member of the TGF-β family, of which the molecular structure had been established (Proc. Natl. Acad. Sci. USA., 81, 371-375, 1989; Progress Growth Factor Research, 1, 267-280, 1989). As the proteins belonging to the same family, activin and others are known (Nature, 231, 776-778, 1986; Nature, 231, 779-782, 1986).

Of BMPs purified from bone matrix, those of types 2 to 8 belong to the TGF-β family. From the information heretofore, these seven types of BMP may be classified into three groups from their molecular similarities; i.e., a group consisting of types 2 and 4, that of type 3, and that of types 5, 6, 7 and 8.

Since BMP is a factor promoting bone induction and production and closely related to developing and healing such bone-tissue disorders as described hereinbefore, determination of BMP in living bodies is essential for precise diagnoses of bone diseases. Moreover, there have long been needs for development of methods to screen for substances similar to or antagonistic against BMP and to purify it more efficiently.

For these purposes, it is generally understood that BMP-specific antibodies should be prepared and applied. However, no such antibody has ever been developed. Since BMP has evolutionally been conserved beyond widely different animal species, antibodies against BMP would hardly be produced. To overcome such a difficulty, development of some other substances than the antibody has been desired.

From intensive studies to search for such substances that are capable of specifically recognizing BMP, the present inventors found out that such problems as described hereinbefore can be solved by preparing and applying a modified BMP receptor.

More particularly, the inventors have for many years carried out studies on genes of BMP (Biochim. Biophys. Res. Commun., 186, 1487-1992; Growth Factors, 7, 233-240, 1992; Growth Factors, 8, 165-172, 1993; Growth Factors, 10, 173-176, 1993; Biochem. J., 298, 275-280, 1993) and those of BMP receptors (Proc. Meetings of Soc. Jpn. Mol. Biol., 2175, 1992; 3209, 1993). With respect to the BMP receptors, no specific one had ever been confirmed; recently, however, a company in the United States and one of the present inventors (Proc. of Int. Symp. Mol. Pharmacol., p 27.1994) isolated and characterized the genes of two types of the BMP receptors (those of BMP types 2 and 4).

BMP, as do other known growth factors, functions by binding to its specific receptor expressed on the cell surface. As a structural characteristic, a receptor generally consists of three domains; extracellular, transmembrane and intracellular domains. As for the BMP receptors, it was confirmed that each of them consists of an extracellular domain rich in cysteine residues, a single-spiked transmembrane domain, and an intracellular domain comprising serine/threonine kinase as commonly conserved among the TGF-β family receptor members.

The present inventors noticed such characteristics that the BMP receptors specifically bind BMP and hypothesized that a modified BMP receptor, comprising an assumably soluble extracellular domain of the receptor and an artificially supplemented epitope tag for discrimination, might be useful for determining BMP, screening for substances that can mimic the biological functions of BMP, and purifying BMP. As the results of intensive studies, the inventors proved that the modified BMP receptor fulfilled such objects and then completed the present invention. Accordingly, the objectives of the invention are to provide a modified BMP receptor comprising an extracellular domain of a BMP receptor and a supplemented epitope tag, the gene of said modified receptor and the prospect of application of said modified receptor.

### DISCLOSURE OF INVENTION

The present invention accomplished to solve such problems as described hereinbefore relates to the gene coding a modified BMP receptor, the modified receptor expressed by the gene and the prospect of application of the modified receptor as described hereinafter.
① The gene of a modified BMP receptor obtained by fusing the gene coding an extracellular domain of the BMP receptor and the gene coding the antigenic determinant recognizable by the specific antibody.
② The gene of the modified BMP receptor mentioned in ① comprising the gene coding an antigenic determinant recognized by the specific anti-oncogene-myc antibody.
③ The gene of the modified BMP receptor comprising the DNA sequence, Sequence No. 1, described hereinafter or a part of said DNA sequence.
④ The modified BMP receptor expressed by the gene specified in ① to ③.
⑤ The modified BMP receptor comprising the amino-acid sequence, Sequence No. 2, described hereinafter or the amino-acid sequence containing said amino-acid sequence.
⑥ The method for BMP determination characterized by the use of the modified BMP receptor described in ④ or ⑤.
⑦ The method for screening for substances similar to or antagonistic against BMP characterized by the use of the modified BMP receptor described in ④ or ⑤.
⑧ The method for purifying BMP characterized by the use of the modified BMP receptor described in ④ or ⑤.
⑨ The bone-disease remedy which contains the modified BMP receptor described in ④ or ⑤ as an active component.

### BRIEF DESCRIPTIONS OF FIGURES

The above and other objects of the invention will be seen by reference to the description taken in connection with the accompanying figures, in which:
Figure 1 schematically illustrates the procedure to subclone myc-smBMPR digested with the restriction enzyme Eco RI into an expression vector pcDNAIamp.
Figure 2 shows, as described in Example 3, the SDS-PAGE profiles of a cultured fluid and a specimen purified thereof on an affinity column of anti-myc antibody.
Figure 3 shows, as described in Example 4, competitive inhibition of BMP activity by a modified BMP receptor.
Figure 4 illustrates, as described in Example 5, the results of determination of BMP by use of the modified BMP receptor.

### BEST MODE FOR APPLYING THE INVENTION

The gene coding the modified BMP receptor of the present invention is a fused gene comprising genes coding an extracellular domain of a BMP receptor and the antigenic determinant that the specific antibody recognizes. Generally, to feasibly and easily distinguish and separate proteins, it is widely known as the epitope-tagging method in which the antigenic determinant (epitope) recognized by the antibody is supplemented to the proteinaceous substance, and then the substance is distinguished and identified with the labeled antibody and the like. Such a method is introduced in books, for example, Method in Enzymology 194, p 509, 1991, wherein the procedure to supplement an antigenic determinant is described in detail. The gene of the invention is one to express a protein comprising an extracellular domain of a BMP receptor and an antigenic determinant recognizable by the specific antibody. Consequently, the protein expressed by the gene may be identified or discrimiated by the antibody.

The antigenic determinant recognized by the antibody as described hereinbefore (hereinafter referred to as epitope tag for convenience) is not limited, but various epitop tags described in the above references are applicable. Examples are as follows: the amino-acid sequence (Glu-Gln-Lys-Leu-Ilu-Ser-Glu-Glu-Asp-Leu) of the antigenic determinant recognized by the anti-oncogene-myc monoclonal antibody (9E10); the amino-acid sequence (Try-Pro-Tyr-Asp-Val-Pro-Asp-Tyr-Ala) of the antigenic determinant recognized by an anti-influenza-hemagglutinin antibody (12CA5). The peptide expressed by the lacZ gene is also applicable, and in such a case the anti-β-galactosidase antibody may be applied.

The gene coding a modified BMP receptor of the invention may be prepared by fusing a gene coding an extracellular domain of the BMP receptor (e.g., a gene digested at the 380th base from a 5' end of the BMP-receptor gene) and a gene coding an epitope tag as described hereinbefore. More concretely, in the case of the antigenic determinant recognized by the anti-oncogene-myc antibody, an example may be given that the gene coding an extracellular domain of the BMP receptor and the gene coding an amino-acid sequence of the antigenic determinant recognized by the anti-myc monoclonal antibody (9E10) are fused by attaching the 5' end of the latter to the 3' end of the former.

The gene coding the BMP receptor may be prepared by its mRNA. Specifically, the mRNA may be extracted from BMP-receptor-expressing organs, tissues, cells and the like by conventional methods including the phenol method, the guanisium-thiocyanate method and the like, and purified by the oligo-dT-cellulose-column method and the like.

A BMP-receptor cDNA may be prepared from the mRNA by conventional methods including the random primer method and the like.

From the amino-acid sequence highly homologous between receptors of TGF-β and activin, PCR primers (oligonucleotides) are synthesized. The cDNA may be amplified by the PCR method with the primers.

The PCR-amplified cDNA may be isolated by such gel electrophoreses as agarose- and acrylamide-gel electrophoreses, followed by extraction. DNA sequences of the extracted cDNA fragments may be determined by conventional methods including the M13 dideoxy method or with a DNA determination kit commercially available. Then, DNA fragments possessing DNA sequences different from those of TGF-β and activin receptors may be selected.

With the DNA fragments as probes, a full-length cDNA coding the BMP receptor may be isolated from a cDNA library, and the DNA sequence of the isolated full-length cDNA may be determined by a conventional method.

The fused gene of the invention comprising the genes coding the extracellular domain of the BMP receptor and the epitope tag may be prepared by integrating the full-length cDNA as prepared hereinbefore into a proper vector, by amplifying the vector by PCR by using proper primers including complementary sequences of the epitope tag and a translation-termination codon, and then by isolating and purifying the amplified gene by a conventional method. Moreover, the fused gene may also be prepared by digesting the full-length cDNA of the BMP-receptor with a proper restriction enzyme, by isolating a cDNA fragment coding the extracellular domain of the receptor, and then by fusing the cDNA fragment and the DNA fragment coding the epitope tag.

The modified BMP receptor of the invention is a fused protein comprising the extracellular domain of the BMP receptor and the epitope tag, and may be prepared by expression of the fused gene comprising the genes coding the extracellular domain of the BMP receptor and the epitope tag by a genetic-engineering method. Such genetic-engineering expression may be conventionally carried out; e.g., by integrating the fused gene comprising the genes coding the extracellular domain of the BMP receptor and the epitope tag or the gene of the restriction-enzyme fragment of the fused gene into a proper expression vector, by inserting the vector to an appropriate host (e.g., bacteria including Escherichia coli and Bacillus subtilis, yeasts, fungi, cultured cells of animal origin and so forth), by transforming the host, by culturing the transformat, and then by extracting the modified BMP receptor from culture supernatant (or supernatant of the crushed cells).

The modified BMP receptor may be purified by conventional methods including dialysis, salting out, ion-exchange chromatography, gel-filtration chromatography and so forth. Moreover, the modified BMP receptor may simply and highly be purified by affinity chromatography on a solid support immobilized with the epitope-tag-recognizing antibody.

The modified BMP receptor may also be prepared by solid-phase peptide synthesis, depending on the amino-acid sequence deduced from the DNA sequence of the modified receptor.

The modified BMP receptor is effective even if a part or parts of the amino-acid sequence are deleted or replaced with other amino acids or another amino-acid sequence is inserted or the N and/or C terminal(s) are supplemented with one or more amino acids, or sugar(s) are also added deleted or replaced, as far as it remains the same function.

The specific reaction occurring between the modified BMP receptor of the invention and BMP was attested by the competitive-reaction method as shown hereinafter.

Namely, it is accepted that MC3T3 cells from a mouse calvaria transform to osteoblasts, that BMP promotes such a process, and that the alkaline-phosphatase (ALPase) activity increases as an indicator of the process (Tampaku Kakusan Koso, 33(2), 186, 1988).

Addition of BMP extracted from bovine bone to the cell culture increased the ALPase activity. However, simultaneous addition of BMP and the modified BMP receptor decreased the ALPase activity with increment of the quantity of the modified BMP receptor added. Consequently, it was attested that the modified BMP receptor and BMP were combined and competitively inhibited the activity of BMP.

The modified BMP receptor of the invention may be characterized by specific binding with BMP through the extracellular domain thereof, and by the specific binding with the antibody through the epitope tag thereof.

By taking advatage of such characteristics of the modified BMP receptor, the method of BMP determination of the invention immunologically determines BMP (quantitatively and qualitatively). For example, BMP may be determined in the following way: a BMP-containing specimen is placed on a microplate, allowed to be absorbed on the plate, and blocked with such a blocking reagent as bovine serum albumin (BSA) if necessary. The modified BMP receptor of this invention is added to react with BMP. The labeled antibody recognizing an epitope tag of the modified BMP receptor is added, and finally the activity of the label of the bound antibody is determined. In place of the labeled antibody as described hereinbefore, a non-labeled primary antibody and thereafter either a labeled secondary antibody or labeled protein A may be applied. The labels used are not essentially limited, but enzymes (e.g., peroxidase, alkaline phosphatase, β-galactosidase and the like), radio isotopes (e.g., ¹²⁵I, ³²P, ³H and the like), fluorescent substances, and so forth used heretofore in the immunological determination may be applied as the labels.

Moreover, the BMP-determination method is not limited to those described hereinbefore, but various other immunological methods including competitive and non-competitive ones, which are commonly known by those skilled in the art.

By the BMP-determination method of the invention, it has been confirmed that the BMP in a range between 1 and 100 ng can quantitatively be determined with rapidity, high sensitivity and high accuracy. The advantage of the BMP-determination method of the invention is as follows: conventional immunological methods can hardly discriminate between precursor and active molecules, whereas the present method can detect each of them as a phisologically significant molecule.

By using the modified BMP receptor of the invention, the BMP-determination method described hereinbefore with a minor modification can also screen for substances physiologically similar to or antagonistic against BMP. For example, if a complex consisting of the modified BMP receptor of the invention and the labeled antibody recognizing the epitope tag is prepared, the complex and a certain test substance are allowed to react, and any reaction occurs between the complex and the test substance, the test substance will be presumed to have physiological properties similar to BMP.

Moreover, since it specifically binds BMP, the modified BMP receptor of the invention can isolate and purify BMP: i.e., the method for BMP purification of the invention relates to the method of affinity chromatography based on such characteristics of the invention.

To explain more specifically by way of example, an insoluble solid support chemically or physically immobilized with an antibody recognizing the epitope tag is prepared and the modified BMP receptor is bound to the prepared solid support. A sample solution containing BMP is allowed to contact with the solid support for BMP to be adsorbed and then BMP adsorbed is eluted with an appropriate solvent to obtain purified BMP. As the insoluble solid support, conventionally used ones, e.g., Sepharose may be used. The antibody is bound to the insoluble solid support by the conventional methods including the formylated cellulose-gel method. The method of BMP purification of the invention may be carried out by either the column or the batch method.

Moreover, the BMP-purification method is not essentially limited to those described hereinbefore, but various other methods known as affinity-column chromatography, which is well known by those skilled in the art, can also be used.

By the BMP-purification method of the invention, BMP can simply, rapidly and highly be purified.

The bone-disease remedy of the present invention contains the modified BMP receptor as an active component. The drug is valuable for treating and preventing BMP-related bone diseases including BMP-producing tumor and the like, since the modified BMP receptor of the invention may specifically and competitively react with BMP as described hereinbefore.

Although the bone-disease remedy may be administered in various pharmaceutical forms (e.g., liquid, solid, and capsule medicine and the like), the drugs are generally prepared as injection or oral drugs consisting of the modified BMP receptor only or comprising the modified BMP receptor and a carrier commonly used. The injection may be prepared by the conventional methods. For example, the modified BMP receptor is dissolved in an appropriate solvent (e.g., sterilized water, buffer, physiological saline and the like), filter-sterilized through a filter membrane and the like, and then dispensed into sterile containers. The oral-drug may be prepared in various forms of, for example, tablet, granule, fine granule, powder, soft or hard capsule, liquid, emulsion, suspension, syrup and the like, which may be prepared by the conventional methods.

The modified BMP receptor contens of the pharmaceutical products may properly be adjusted depending on the forms of the drug, indications treated and so forth.

In preparing the drugs, a stabilizer may be added. The stabilizers are, for example, albumin, globulin, gelatin, glycine, mannitol, glucose, sorbitol, ethyleneglycol and the like. Moreover, the pharmaceutical preparations of the invention may contain additives requisite to prepare the drugs; e.g., excipient, solubilizing aid, antioxidant, soothing agent, isotonicity aid and the like. In case of liquid drug, frozen storage or storage by lyophilization and the like are desirable. The lyophilized drug may be rehydrated by adding distilled water for injection and the like immediately before use.

Bone-disease remedy of the invention may properly be administered by various routes suitable for the forms of the drug preparation. Dosage of the drug may properly be adjusted depending on the symptoms, age, body weight and the like of the patient.

### INDUSTRIAL APPLICABILITY

According to the present invention, the gene coding a modified BMP receptor is presented and the modified BMP receptor may efficiently be prepared by expression by the gene.

By using the prepared modified BMP receptor, BMP can simply and precisely be determined, substances physiologically similar to or antagonistic against BMP can be screened for, and BMP can rapidly and highly be purified. Moreover, since the modified BMP receptor specifically binds BMP, the modified BMP receptor is useful as a bone-disease remedy for therapy and prevention of various bone diseases associated with BMP. Consequently, the modified BMP receptor of the invention may broadly be applied for purification of BMP, as a diagnostic reagent for various BMP-associated diseases, as a reagent for research, as a medicine, and so forth.

### EXAMPLES

The present invention will specifically be explained by examples hereinafter, which should not be construed as limiting the scope of the present invention.

### Example 1

### Preparation of the mouse BMP receptor

Depending on the amino-acid sequences of intracellular-kinase domains VIII and XI, of which the receptors are most homologous between TGF-β and activin (Thr-Met-Ala-Pro-Glu-Val and Glu-Cys-Trp-Asp-His-Asp, respectively), two oligonucleotides were synthesized as the following:
Synthetic oligonucleotide 1: 5'-TA(TC)ATGGC(TCAG)CC(TCAG)GA(AG)GT-3'
Synthetic oligonucleotide 2: 5'-(AG)TC(AG)TG(AG)TCCCA(AG)CA(TC)TC-3'

These oligonucleotides were synthesized by a Milligen-Biosear apparatus by the conventional method.

Next, according to a method described in a reference (Anal. Biochem., 162, 156-157, 1987), the whole RNA molecule was isolated and purified from MC3T3-E1 cells which are known to transform to osteoblasts upon reacting to BMP.

Then, by the conventional method (the random-primer method), cDNA was synthesized with the RNA obtained as a template.

By the PCR method described in references (Science 230, 1350-1354, 1985 and others), the DNA fragments similar to the receptors of TGF-β and activin as described hereinbefore were amplified with cDNA and the synthetic oligonucleotides as primers.

The base sequences of the amplified DNA fragments were determined, and the DNA fragment possessing base sequences different from those of TGF-β and the activin receptors were identified.

Finally, with the DNA fragment as a probe, a full-length gene coding the mouse BMP receptor aimed at was isolated from a mouse cDNA library. Of the clones obtained, a 3.9-kb clone designated mTFR11 was proved to be the gene of the mouse BMP receptor.

### Example 2

### Preparation of Xenopus laevis BMP receptor

In the same manner as described in Example 1, a DNA fragment was identified with the whole RNA prepared from early-stage embryos of *Xenopus laevis* as a templet, and designated xTFR11. With the DNA fragment as a probe, a full-length DNA coding the *Xenopus laevis* BMP receptor was isolated from a cDNA library of the stage 3-5 embryos of *Xenopus laevis.*

### Example 3

### Preparation of the gene of the modified BMP receptor and expression thereof

The following oligonucleotides were synthesized by the same method as that used in Example 1.
Synthetic oligonucleotide 3: 5'-TGAATTCCTACAGGTCCTCCTCGGAGATCAGCTTCTGCTCTCGGATGCTGCCATCAAAG-3'
Synthetic oligonucleotide 4: 5'-CAGGAAACAGCTATGAC-3'

Next, the cDNA of mTFR11 prepared in Example 1 was subcloned into a Bluescript KS(-) vector and amplified by the PCR method with this vector and the oligonucleotides 3 and 4. A DNA fragment consisting of a DNA sequence coding the extracellular domain of the BMP receptor and the other sequences of 5'-GAGCAGAAGCTGATCTCCGAGGAGGACCTG-3' coding an amino-acid sequence (Glu-Gln-Lys-Leu-Ile-Ser-Glu-Glu-Asp-Leu) recognized by the anti-oncogene-myc antibody(9E10) and the protein-translation-termination codon (TAG), which were designed to be fused at a 3' end of the extracellular domain of the BMP-receptor, was amplified. Then, the DNA sequence thereof (designated myc-smBMPR) was determined and is shown in sequence No.1, in which the underlined part shows the sequence coding the epitope tag.

The amino-acid sequence deduced from the DNA sequence of the modified BMP receptor is shown in Sequence No.2.

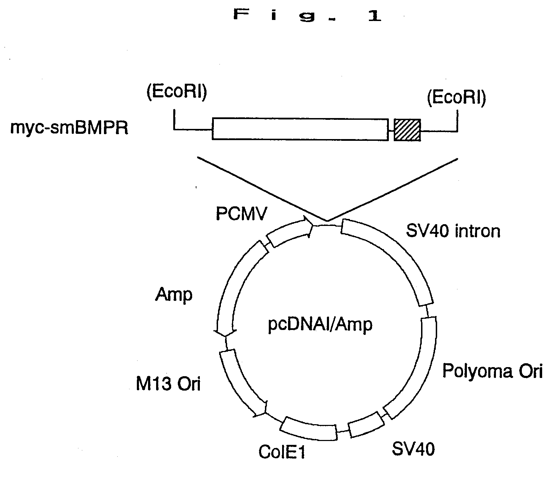

After digesting the DNA sequence of the modified BMP receptor with the restriction enzyme Eco RI, its fragment was subcloned into an expression vector for the animal-cell host, pcDNAIamp. The outline of the procedure is shown in Fig. 1.

Next, the modified BMP receptor of the invention was obtained by transforming COS7 cells with the expression vector by the DEAE-dextran method and by expressing them.

More specifically, the COS7 cells (1 x 10⁸) were cultured in Dulbecco's modified medium (dish area: 6,000 cm²). A 10-µg portion of the DNA mixed with 3 ml DEAE-dextran was added to the cells, which were allowed to stand for 15 min at room temperature, and washed. Then, the cells were cultured in 10% serum-containing Dulbecco's modified medium for one day and cultured for furthur three days after medium exchange with a serum-free one. Thereafter, culture fluid (5 litter in total) was recovered, filtrated through a 10-kDa ultrafilter (Asahikasei). The filtrate was mixed with the same volume of Tris buffer (20 mM Tris-HCl, pH 7.4, 0.15 M NaCl, referred to as TBS buffer hereinafter), and loaded on an affinity column (5 ml), of which the solid support (AffiGel) had been immobilized with the anti-oncogene-myc monoclonal antibody (9E10).

The adsorbed fraction was eluted with a citrate buffer, pH 3.0. After dialysing the eluted solution against Tris buffer, the modified BMP receptor of the invention was obtained and stored at 4°C.

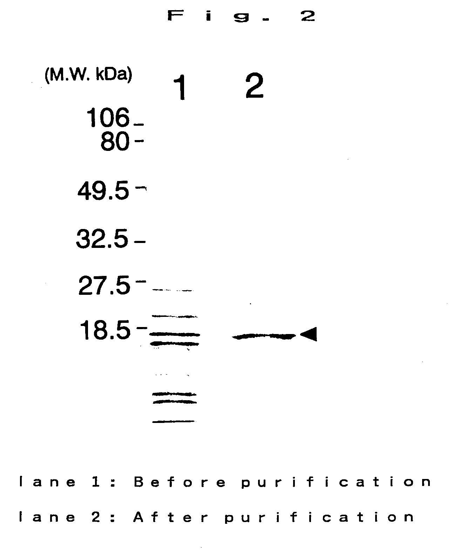

The SDS-PAGE profiles of the specimens before and after affinity-column purification are shown in Fig. 2. As compared with the specimen before affinity chromatography, the fraction adsorbed on the column and eluted thereafter consisted solely of a 18-kDa protein, being identical to the theoritical molecular weight of the modified BMP receptor. From these observations, it was attested that the modified BMP receptor of the invention may have been purified by the antibody-immobilized affinity chromatography.

### Example 4

### Determination of biological activity

To each well of a 48-well microplate, 10⁴ MC3T3-E1 cells were inocultaed (medium:10% FBS-containing αMEM). After incubation for two days, the culture medium was exchanged with a medium containing 10 ng/ml BMP. After addition of 10 or 50 ng/ml of the modified BMP receptor prepared in Example 1 and incubation for further 48 h, alkaline phosphatase (ALPase) activities were determined and compared among test variables with and without addition of BMP.

Determination of the ALPase activity was carried out in the following way: the culture medium was removed from the microplate, the wells was washed with a phosphate buffer, a color-developing substrate (0.56 M 2-amino-2-methylpropane-1-ol, 1 mM MgCl₂, and 10 mM sodium *p*-nitrophenylphosphate) was added to each well, and the absorbance at 405nm was read after one-hour reaction.

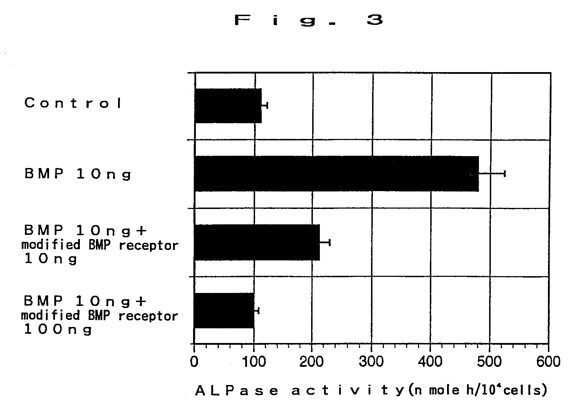

The results are shown in Fig. 3. As shown in Fig. 3, the biological activity of BMP was inhibited by addition of the modified BMP receptor.

### Example 5

### Carrying out of ELISA

The BMP ranging from 10 to 100 ng in TBS buffer was adsorbed to a 96-well microplate for 12 h at 4°C. After washing and blocking with 5% BSA, a 200-ng portion of the modified BMP receptor prepared in Example 1 was added to each well, and allowed to react for 2 h at room temperature. The supernatant was removed and the precipitate was washed.

Then, a 100-ng portion (in TBS buffer containing 1% BSA and 0.1% Tween 20) of the monoclonal antibody against oncogene myc (9E10) was added to each well as a primary antibody. The mixture was allowed to react for an hour at room temperature, the suppernatant was removed and the precipitate was washed. A 100-ng portion of horse-radish-peroxidase-conjugated anti-IgG antibody (in TBS buffer containing 1% BSA and 0.1% Tween 20) was added to each well as the secondary antibody, allowed to react for another hour at room temperature, the supernatant was removed and the precipitate was washed. After addition of a color-developing reagent, 0.1% *o*-phenylenediamine (in 0.1 M citrate buffer, pH 4.5), absorbance at 490nm was read.

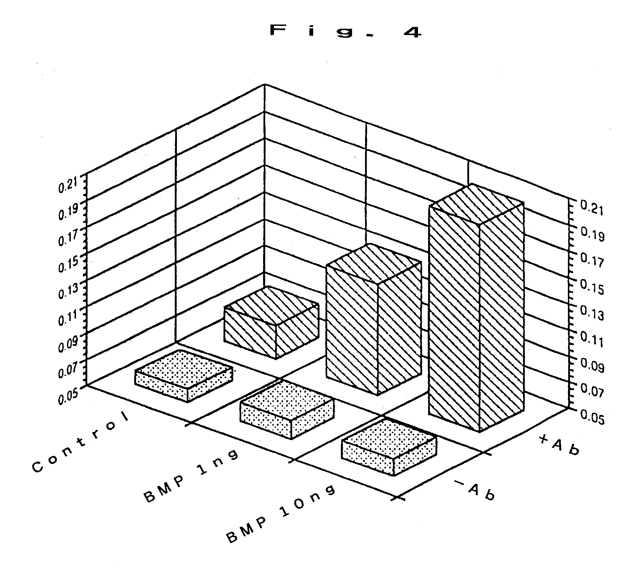

The results are shown in Fig. 4, where an abbreviation Ab means a primary antibody. As shown in Fig. 4, it was attested that the modified BMP receptor of the present invention quantitatively determines BMP.

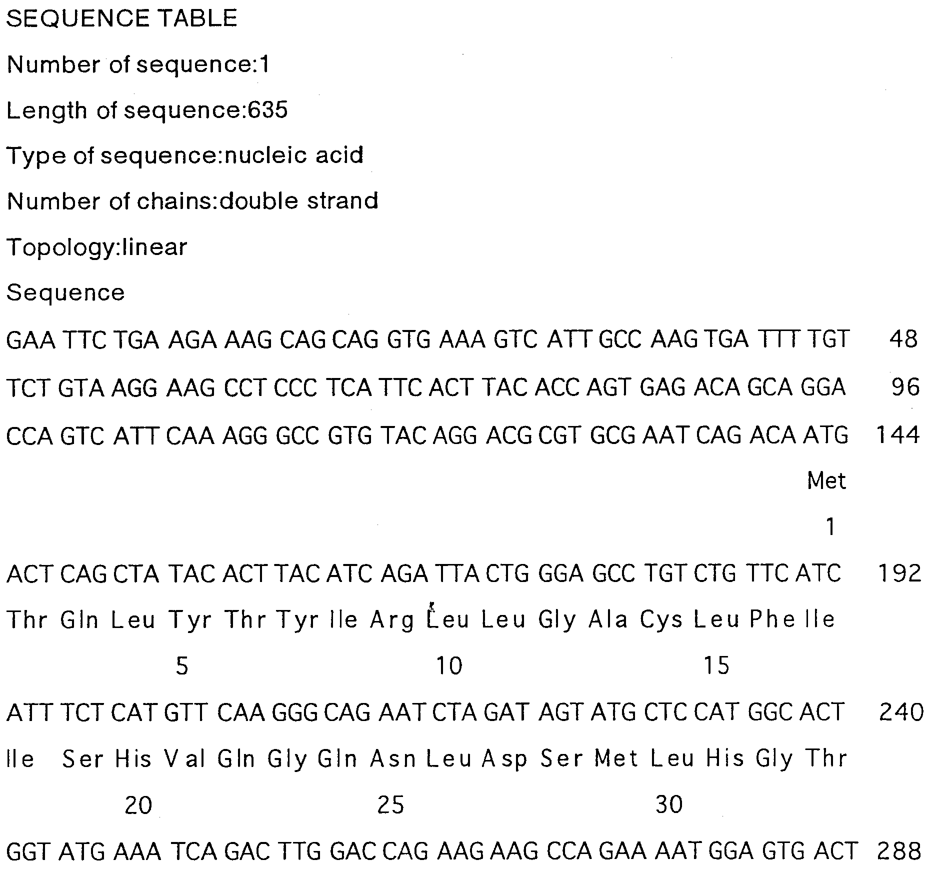

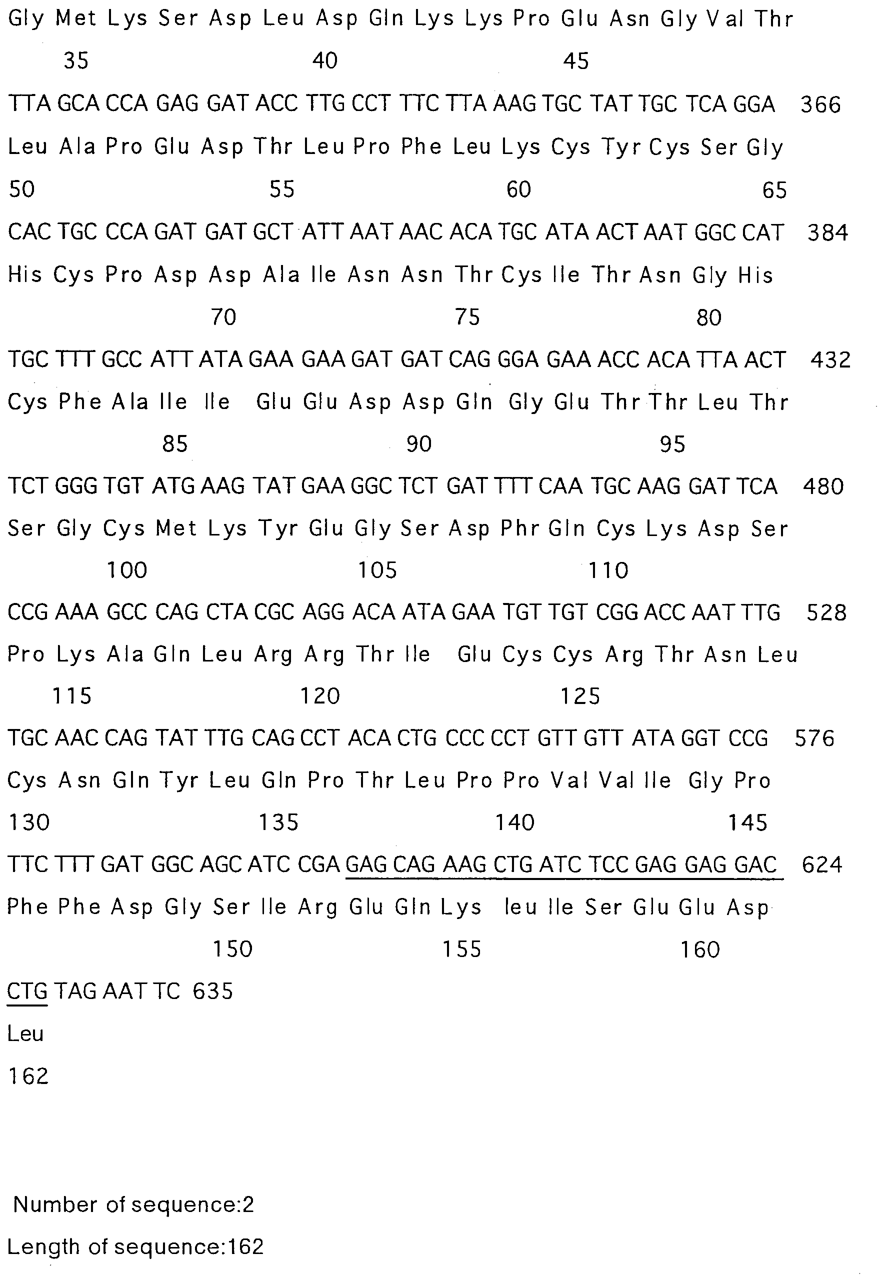

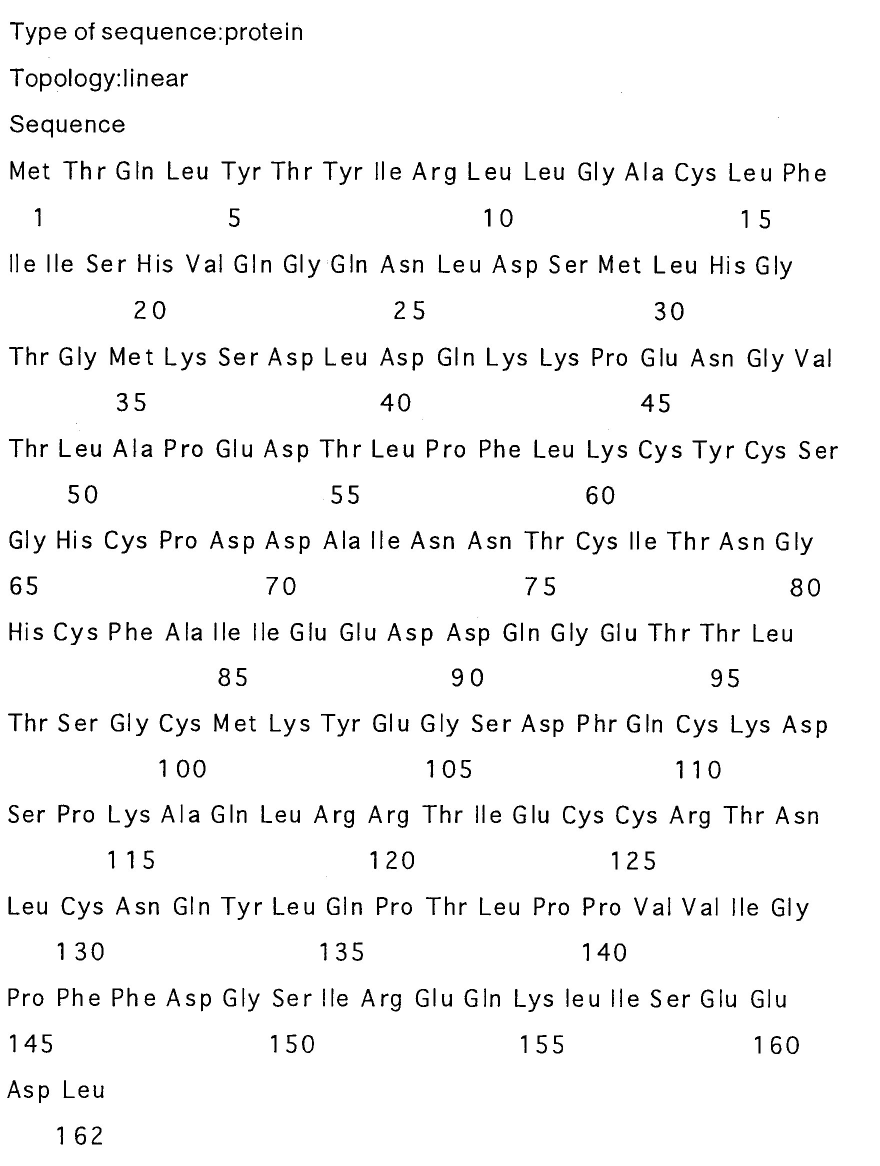

## Claims

1. A gene of the modified bone-morphogenetic-protein (BMP) receptor obtained by fusing a gene coding an extracellular domain of the BMP receptor and a gene coding an antigenic determinant recognized by a specific antibody.

2. The gene of the modified BMP receptor as claimed in claim 1, wherein the gene coding an antigenic determinant recognized by the antibody is the one coding the antigenic determinant recognized by the anti-oncogene myc antibody.

3. A gene of the modified BMP receptor comprising the DNA sequence defined by Sequence No. 1 or a part of said DNA sequence.

4. A modified BMP receptor expressed by the gene as claimed in claim 1, 2 or 3.

5. A modified BMP receptor comprising the amino-acid sequence defined by Sequence No. 2 or the amino-acid sequence containing said amino-acid sequence.

6. A method of BMP determination, characterized by the use of the modified BMP receptor as claimed in claim 4 or 5.

7. A method of screening for substances similar to or antagonistic against BMP, characterized by the use of the modified BMP receptor claimed in claim 4 or 5.

8. A method of purification of BMP, characterized by the use of the modified BMP receptor as claimed in claim 4 or 5.

9. A bone-disease remedy which contains the modified BMP receptor as claimed in claim 4 or 5 as an active component.
